# EUROPÄISCHE PATENTSCHRIFT

(11) **EP 1 592 140 B1**
(45) Veröffentlichungstag und Bekanntmachung des Hinweises auf die Patenterteilung: **15.04.2009**
(21) Anmeldenummer: 05100909.0
(22) Anmeldetag: 09.02.2005
(51) Int. Cl.: H04B 1/16, H04H 40/18, H03D 7/16

(54) **Vorrichtung für den Empfang von Rundfunksignalen**
Device for the reception of broadcast signals
Dispositif pour la réception de signaux de radiodiffusion

(30) Priorität: 27.04.2004 DE 102004020551
(43) Veröffentlichungstag der Anmeldung: 02.11.2005
(73) Patentinhaber: ROBERT BOSCH GMBH, 70442 Stuttgart (DE)
(72) Erfinder: Passoke, Jens, 30539, Hannover (DE)

(56) Entgegenhaltungen:
- EP-A- 0 959 559
- EP-A- 1 011 203
- GB-A- 2 392 566
- BOLLE M ET AL: "The receiver engine chip-set for digital audio broadcasting" SIGNALS, SYSTEMS, AND ELECTRONICS, 1998. ISSSE 98. 1998 URSI INTERNATIONAL SYMPOSIUM ON PISA, ITALY 29 SEPT.-2 OCT. 1998, NEW YORK, NY, USA,IEEE, US, 29. September 1998 (1998-09-29), Seiten 338-342, XP010316821 ISBN: 0-7803-4900-8

## Beschreibung

Die vorliegende Erfindung betrifft eine Vorrichtung für den Empfang von Rundfunksignalen, wobei die Vorrichtung zum Empfang mindestens eines digital übertragenen Rundfunksignals und zum Empfang mindestens eines analog oder digital und analog gemischten übertragenen Rundfunksignals vorgesehen ist und zur Umsetzung des mindestens einen analog übertragenen Rundfunksignals auf eine Zwischenfrequenz Signale verwendet werden, die von Empfängerkomponenten wie beispielsweise der Signalquelle des Mischoszillatorsignals ausgegeben werden, die zur Frequenzumsetzung des mindestens einen digital übertragenen Rundfunksignals vorgesehen sind. Im Falle des IBOC in den USA ist ein hybrider Mode vorgesehen, bei DRM ist ein digitaler Standard bei den Frequenzen der herkömmlichen analogen Amplitudenmodulation, also zwischen 153kHz und 26,5MHz, vorgesehen.

### Stand der Technik

Aus der Veröffentlichung "D-Fire 2: A combined DAB/FM Receiver System on Chip" von Clawin, Gieske, Hofmann, Kupferschmidt, Mlasko, Naberfeld, Passoke, Spreitz und Stepen, veröffentlicht auf der Ausstellung "Micro Tech 2000" vom 25. Bis 27. September 2000, ist insbesondere aus Kapitel 3 ein Empfängerkonzept zum Empfang von DAB-Rundfunk bekannt, das für die L-Band-Signale im Bereich von 1452 bis 1492 MHz und für die III-Band-Signale im Bereich von 170 bis 240 MHz einen kompletten Signalpfad zur Verfiigung stellt, wobei hierzu automatische Hochfrequenzverstärkungsregelungen, zwei Mischer, Zwischenfrequenzverstärker mit automatischer Verstärkungsregelung sowie die notwendigen Phasenregelschleifen auf einem einzigen Chip realisiert sind. Die Designziele waren gleichermaßen kleine Abmessungen, eine günstige Herstellung sowie ein sparsamer Energieverbrauch.

Aus der EP 1 011 203 A1 ist ein Empfänger und ein Empfangsverfahren für Rundfunksysteme bekannt, bei denen analoge und digitale Rundfunksignale in benachbarten Frequenzbändern übertragen werden. Der Empfänger weist einen analogen Verarbeitungsschaltkreis auf, der analoge Rundfunksignale empfängt, verarbeitet und Audiosignale extrahiert, weiterhin weist der Empfänger einen digitalen Verarbeitungsschaltkreis auf, der digitale Rundfunksignale empfängt, verarbeitet und Audiosignale extrahiert. Weiterhin ist ein Umschalteschaltkreis vorgesehen, der entweder Audiosignale auswählt, die der analoge Verarbeitungsschaltkreis oder der digitale Verarbeitungsschaltkreis zur Verfügung stellt, sowie eine Steuerschaltung, die die Energieversorgung des digitalen Verarbeitungsschaltkreises steuert, sowie Erkennungsmittel, die erkennen, ob eine digitale Rundfunkstation ausgewählt wurde. Die Steuerschaltung versorgt den digitalen Verarbeitungsschaltkreis nur dann mit Energie, wenn das Erkennungsmittel erkennt, dass eine digitale Rundfunkstation ausgewählt wurde, wodurch der Energieverbrauch des Empfängers reduziert werden kann.

### Kern und Vorteile der Erfindung

Der Kern der vorliegenden Erfindung ist es, ein Empfängerkonzept gemäß Anspruch 1 anzugeben. Vorteilhafte Weiterbildungen und Ausgestaltungen ergeben sich aus den Unteransprüchen.

Vorteilhafterweise wird das digital übertragene Hörfunksignal auf zwei unterschiedlichen Frequenzbändern verteilt übertragen wird. Im Falle des Übertragungsstandards DRM sind dabei die LW-, MW- und alle KW-Bänder betroffen.

Weiterhin ist es vorteilhaft, dass das digital übertragene Hörfunksignal nach mindestens einem der Übertragungsstandards Digital Audio Broadcast (DAB), Digital Radio Mondiale (DRM), Digital Multimedia Broadcast (DMB) sowie Digital Video Broadcast Terrestrisch (DVB-T) übertragen wird.

Weiterhin ist es vorteilhaft, dass das analog übertragene Hörfunksignal frequenzmodulierter Hörfunk (FM), insbesondere bei Trägerfrequenzen zwischen 88 MHz und 108 MHz und/oder amplitudenmodulierter Hörfunk (AM), insbesondere bei Trägerfrequenzen zwischen 520 kHz und 1710 kHz ist. Die Erfmdung bezieht sich aber auch auf den Empfang entsprechender FM-Frequenzen in Japan etwa zwischen 76 bis 90 MHz, sowie aller AM-Frequenzen zwischen etwa 150kHz und 26,5 MHz. Dabei können die Signale in allen Bändern digital oder analog oder in einer hybriden Modulation vorliegen und empfangen werden.

Weiterhin ist es vorteilhaft, dass Komponenten zur Verarbeitung des digital übertragenen Rundfunksignals, des frequenzmoduliert übertragenen Rundfunksignals und des amplitudenmoduliert übertragenen Rundfunksignals wie beispielsweise LNA(Low Noise Amplifier), Mischer, ZF-Verstärker und/oder ZF-Filter auf einem Chip monolithisch integriert sind.

Weiterhin ist es vorteilhaft, dass ein analoger Zwischenfrequenzverstärker vorgesehen ist, der zur Ausgabe des digital übertragenen Rundfunksignals, des frequenzmoduliert übertragenen Rundfunksignals und des amplitudenmoduliert übertragenen Rundfunksignals vorgesehen ist. Dabei kann dieser die Signale alternativ oder gleichzeitig verstärken.

Vorteilhafterweise ist das ZF-Filter eines oder mehrer Signalpfade mit einer Mischstufe integriert, wobei die nachfolgenden signalverarbeitenden Stufen entsprechend auszulegen sind.

Weiterhin ist es vorteilhaft, dass für mindestens zwei Mischstufen unterschiedlicher Signalpfade das Mischsignal aus mindestens einem gemeinsamen Oszillator abgeleitet wird.

Weiterhin ist es vorteilhaft, dass bei dem die dem ZF-Verstärker nachfolgende signalverarbeitende Stufe ein auf dem Chip monolithisch integrierte Analog/Digital-Wandler ist. Dabei kann ein A/D-Wandler beispielsweise zwei Signale unterschiedlicher Zwischenfrequenzen gleichzeitig umsetzen, so dass mit einem solchen Konzept beispielsweise gleichzeitig ein DAB-Empfang und ein TMC-Datenempfang möglich ist.

### Zeichnung

Nachfolgend wird ein Ausführungsbeispiel der Erfindung anhand einer Zeichnung erläutert. Diese zeigt ein schematisches Blockschaltbild der erfindungsgemäßen Vorrichtung.

### Beschreibung des Ausführungsbeispiels

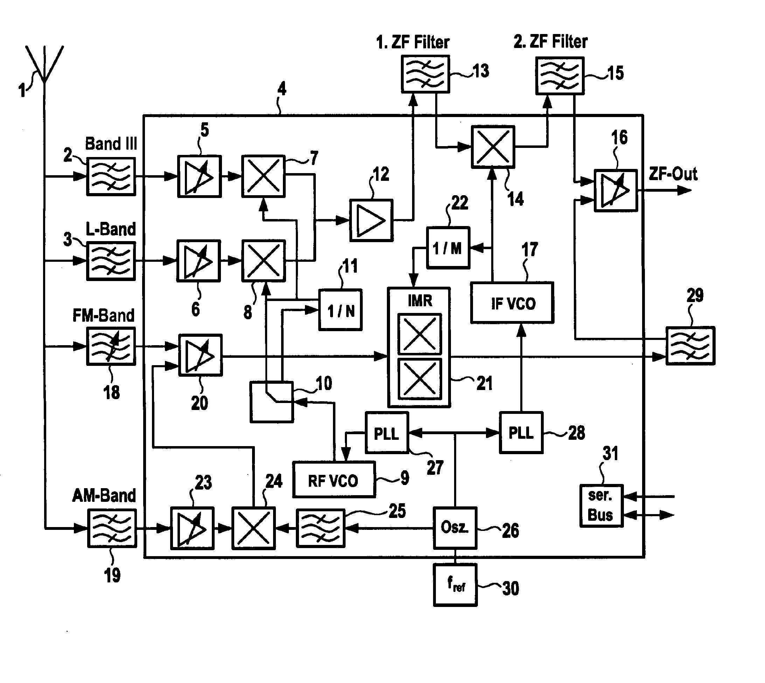

In der Figur ist ein schematisches Blockdiagramm eines kombinierten Empfängers dargestellt, der beispielsweise DAB-Signale, FM-Signale und AM-Signale mittels eines einzigen, kombinierten Empfangskonzepts empfangbar und ausgebbar ist. Gegenüber dem Empfangskonzept aus dem Stand der Technik weist die Figur einen zusätzlichen FM-Pfad auf, der einen rauscharmen Verstärker (LNA) als automatische Verstärkerregelung (AGC) 20 und Mischer 21 aufweist. Hieran schließt sich ein Zwischenfrequenz (ZF)-Filter 29 sowie ein Zwischenfrequenz (ZF)-Verstärker 16 an. Dabei kann die Generierung des Oszillatorsignals, das dem Mischer 21 vom Frequenzteiler 22 zugeführt wird, aus dem Zwischenfrequenzoszillatorsignal des Zwischenfrequenzoszillators 17 des DAB-Signalpfades gewonnen werden, wenn es mittels des Frequenzteilers 22 in der Frequenz im Verhältnis 1:M heruntergeteilt wird. Damit ist für die Integration des frequenzmodulierten Signalpfades (FM-Pfad) keine zusätzliche Phasenregelschleife (PLL) erforderlich. Die Integration des Signalpfades für amplitudenmodulierte Empfangsfrequenzen erfolgt mit Hilfe eines zusätzlichen Mischers 24. Mit Hilfe des Mischers 24 wird das empfangene AM-Band in einen höheren Frequenzbereich, nämlich das FM-Band, umgesetzt, so dass für die weitere Signalverarbeitung der AM-Empfangssignale der oben beschriebene FM-Signalpfad bis zum analogen Zwischenfrequenzausgang (ZF-Out) genutzt werden kann. Dabei ergibt sich, dass bei Nutzung des gesamten AM Frequenzbereichs der Abstimmbereich des FM-Signalpfades erweitert werden kann oder das Mischsignal mit einem umschaltbaren Frequenzteiler entsprechend geteilt wird, so dass das Mischerausgangssignal innerhalb eines der weltweiten FM-Empfangsbänder liegt.

Im einzelnen zeigt die Figur eine Antenne 1, die im dargestellten Beispiel als kombinierte Antenne für alle empfangenen Bände ausgeführt sein kann. Weiterhin ist es aber auch möglich, für einzelne Bänder getrennte Antennen vorzusehen und die empfangenen Signale den einzelnen Empfangsfiltern 2, 3, 18, 19 getrennt oder in Summe zuzuführen. Das Antennenausgangssignal wird zum einen einem Empfangsfilter 2 für das Band III zugeführt, der eine Vorselektion der Empfangssignale vornimmt. Das Ausgangssignal des Band III-Filters 2 wird einem Eingang der DAB-/FM-/AM-Empfangsvorrichtung zugeführt. Weiterhin wird das Antennenausgangssignal einem Empfangsfilter 3 für das L-Band zugeführt, das ebenfalls eine Vorselektion der Empfangsfrequenzen vornimmt und das Ausgangssignal dieses Empfangfilters 3 einem weiteren Eingang der Empfangsvorrichtung 4 zuführt. Die Ausgangssignale der Empfangsfilter 2 bzw. 3 werden automatisch den Verstärkerregelungen (AGCs) 5 bzw. 6 zugeführt, die beispielsweise als rauscharme Verstärker (LNAs) ausgeführt sein können. Die verstärkten Ausgangssignale der Verstärkerregelung 5, 6 werden einem Mischer 7 für das III-Band sowie einem Mischer 8 für das L-Band zugeführt. Die Mischer 7, 8 erhalten als weiteres Eingangssignal das Ausgangssignal eines Hochfrequenzoszillators 9 (RF-VCO), das wahlweise mittels des Umschalters 10 direkt auf die Mischer 7, 8 gegeben werden kann oder erst mittels des Frequenzteilers 11 im Verhältnis 1:N den Mischern 7, 8 zuführbar ist. Bei entsprenchender Dimensionierung bzw. Einstellung des Frequenzteilers 11 kann das Band III aufwärts und das L-Band abwärts auf eine gemeinsame Zwischenfrequenz gemischt werden. Hierdurch ist eine Ausweitung eines solchen Empfangskonzeptes auf weitere Empfangsfrequenzen denkbar.

Die Ausgangssignale der Mischer 7, 8 werden einem gemeinsamen Zwischenverstärker 12 zugeführt, bevor das Ausgangssignal des Zwischenverstärkers 12 einem extern angeordneten ersten Zwischenfrequenzfilter 13 zugeführt wird. Eine Variante ist die Integration des Zwischenfrequenzfilters unter Verwendung aktiver Filterschaltungen, eine weitere Variante ist der Verzicht auf dieses Filter, wobei dann die nachfolgenden signalverarbeitenden Stufen entsprechend großsignalfest ausgelegt werden müssen. Danach wird das Ausgangssignal des ersten Zwischenfrequenzfilters 13 einem Zwischenfrequenzmischer 14 zugeführt wird, der als weiteres Mischereingangssignal das Ausgangssignal einer Einheit 17 zur Aufbereitung eines zweiten Zwischenfrequenzsignals (IF-VCO) erhält, und das Ausgangssignal des Zwischenfrequenzmischers 14 einem wiederum extern angeordneten oder integrierten aktiven zweiten Zwischenfrequenzfilter 15 zuführt. Wird hier das zweite Zwischenfrequenzfilter eingespart, so muss der nachfolgende Signalpfad in seinem Pegelplan entsprechend ausgelegt werden. Das Ausgangssignal dieses zweiten, internen oder externen Zwischenfrequenzfilters 15 wird einem Zwischenfrequenzausgangsverstärker 16 zugeführt, bevor das Ausgangssignal dieses Ausgangsverstärkers 16 als Zwischenfrequenzausgangssignal (ZF-Out) für die weitere Verarbeitung zur Verfügung steht. Sowohl der Hochfrequenzoszillator 9 (RF-VCO) als auch der Zwischenfrequenzoszillator 17 (IF-VCO) werden mittels einer Frequenz f_{ref} eines Referenzoszillators 30, der extern angeordnet sein kann, gesteuert. Diese Referenzfrequenz f_{ref} wird einem Oszillator 26 zugefiihrt, mittels dem die erste Phasenregelschleife 27 (PLL) sowie die zweite Phasenregelschleife 28 (PLL) gesteuert werden. Der Ausgang der ersten Phasenregelschleife 27 wird hier bei dem Hochfrequenzoszillator 9 (RF-VCO) zugeführt und das Ausgangssignal der zweiten Phasenregelschleife 28 dem Zwischenfrequenzoszillator 17 (IF-VCO) zugeführt.

Mittels der Antenne 1 werden weiterhin Signale des FM-Bandes empfangen, die dem FM-Band-Empfangsfilter 18 zugeführt werden. Dieser FM-Band-Empfangsfilter 18 nimmt eine Vorselektion der Empfangsfrequenzen vor und führt die vorselektierten Signale einem FM-Eingang der Empfangsvorrichtung 4 zu, in der dieses Signal einer automatischen Verstärkerregelung 20 für das FM-Band zugeführt wird. Nach der Vorverstärkung mittels der automatischen Verstärkerregelung 20 wird das FM-Signal einem Mischer 21 zugeführt, in dem das FM-Signal auf eine Zwischenfrequenz umgesetzt wird. Hierzu wird dem Mischer 21 das Ausgangssignal des Zwischenfrequenzoszillators 17 zugeführt, das über einen Frequenzteiler 22 im Verhältnis 1:M heruntergeteilt wird und dem Mischer 21 zugeführt wird.

Im dargestellten Ausführungsbeispiel ist der Mischer 21 als Spiegelunterdrückungsmischer (IMR-Mischer) ausgeführt, jedoch kann der Mischer 21 als einfacher Mischer ausgeführt sein. Durch die Ausführung des Mischers 21 als IMR-Mischer ergibt sich der Vorteil, dass weniger externe Bauteile benötigt werden, wodurch eine platzsparendere und kostengünstigere Konstruktion erreicht wird.

Das Ausgangssignal des Mischers 21 wird einem extern angeordneten Zwischenfrequenzfilter für das FM- und AM-Band 29 zugeführt, mittels dem das FM-Band durchstimmbar ist. Das Ausgangssignal dieses Zwischenfrequenzfilters 29 wird wieder dem Zwischenfrequenzausgangsverstärker 16 zugeführt, der ein Zwischenfrequenzausgangssignal für die weitere Verarbeitung zur Verfiigung stellt.

Für den Empfang von amplituden- und/oder auch digital modulierten Empfangssignalen im AM-Band, wie beispielsweise beim system IBOC in den USA, das einen hybriden Mode bei MW-Frequenzen aufweist, werden bereits bestehende Signalpfade benutzt, indem das Empfangssignal im AM-Frequenzbereich von der Antenne 1 über ein AM-Band-Empfangsfilter 19 vorselektiert wird und einer automatischen Verstärkerregelung (AGC) für das AM-Band zugeführt wird, die beispielsweise als rauscharmer, regelbarer Verstärker (LNA) ausgeführt sein kann. Das Ausgangssignal dieser automatischen Verstärkerregelung 23 wird einem Mischer 24 zugeführt, indem das AM-Band-Signal in das FM-Band umgesetzt wird. Hierzu wird dem Mischer 24 das Ausgangssignal des Oszillators 26 zugeführt, das über ein Mischerfrequenzfilter 25 gefiltert wird, so dass die Umsetzfrequenz für den Mischer 24 zur Verfiigung steht. Das Ausgangssignal des Mischers 24 ist nun ein Signal, das im FM-Band liegt, so dass dieses Signal mittels des FM-Empfangspfades weiterverarbeitet werden kann, indem das Ausgangssignal des Mischers 24 dem Verstärker 20, der als automatische Verstärkerregelung für das FM-Band in Form eines rauscharmen Verstärkers (LNA) ausgeführt sein kann, erfolgt. Durch die Umsetzung der AM-Signale in das FM-Band kann man sowohl die Signale bei AM-Frequenzen als auch die Empfangssignale bei FM-Frequenzen mittels eines einzigen Empfangspfades verarbeiten, was bei einer möglichen Ausweitung von DAB-Übertragungsspektren auf FM-Frequenzen vorteilhaft ist. Ebenso ist es möglich, Schaltungsteile für das III-Band bzw. das L-Band, die beispielsweise für digitale Rundfunkverfahren wie DAB oder DRM verwendet werden, auch für die Empfangssignale bei FM- und bei AM-Frequenzen mitzuverwenden. Weiterhin ist eine Schnittstelle für eine Steuereinrichtung 31 vorgesehen, mittels der die einzelnen Empfangsparameter eingestellt werden können, sowie Verstärkereinstellungen oder Umschalter für Frequenzteiler entsprechend eingestellt werden können.

Es sind auch weitere Varianten des dargestellten Ausführungsbeispiels möglich, indem beispielsweise ein oder mehrere Analog-/Digital-Wandler integriert werden. Ebenso ist es nicht zwingend notwendig, dass das Zwischenfrequenzsignal des FM-Signalpfades nach dem Mischvorgang im Mischer 21 addiert wird, sondern dieses kann auch mittels zwei Analog/Digital-Wandlern zugeführt werden. Ebenso kann die Verwendung eines externen Zwischenfrequenzfilters 13, 15 eingespart werden, wobei bei geringerer Selektion des Zwischenfrequenzfilters die Mischstufe 14 entsprechend großsignalfester ausgelegt werden muß und ein nachfolgender Analog-/Digital-Wandler vorgesehen werden. Für die Herstellung der Schaltung für den DAB-Empfang ist es vorteilhaft, eines der beiden oder beide Zwischenfrequenzfilter 13, 15 mitzuintegrieren oder gegebenenfalls einzusparen und diese auch für den FM- und AM-Empfang zu benutzen. Ein wesentlicher Aspekt sind hier die Kosten für die verwendeten Filter. Die Integration und insbesondere die Einsparung bedeuten, daß der nachfolgende Signalpfad entsprechend angepaßt werden muß.

Der oder die integrierten Analog/Digital-Wandler folgen nach dem Zwischenfrequenzverstärker des jeweiligen Signalpfades. Diese können beispielsweise auf einem seperaten IC oder in dem Backend integriert werden. Vorteil der Integration des Wandlers auf dem Frontend ist unter anderem, daß die Demodulation und oder Dekodierung des Signals auf einem Baustein erfolgen könnte, der softwarekonfigurierbar ist, beispielsweise mittels eines Digitalen Signalprozessors. Eine solche Lösung bietet eine hohe Flexibilität und kann deutlich kostengünstiger sein. Gründe hierfür sind unter anderem die Verwendung von Standardbausteinen sowie der Vorteil, daß keine oder wenig Chipfläche exklusiv für bestimmte Übertragungsstandards vorgehalten werden müssen. So kann mittels der Rechenleistung, die in Europa für DAB-Dekodierung genutzt wird diese in den USA für die IBOC-Dekodierung oder weltweit für DRM-Dekodierung genutzt werden.

## Patentansprüche

1. Vorrichtung für den Empfang von Rundfunksignalen zweier oder mehrerer Übertragungsstandards, die teilsweise in unterschiedlichen Frequenzbändern ausgestrahlt werden und die in voneinander unabhängigen Signalpfaden (5 bis 16; 6 bis 16; 20 bis 16; 23 bis 16) verarbeitet werden, wobei die Vorrichtung zum Empfang mindestens eines digital übertragenen Rundfunksignals und zum Empfang mindestens eines analog übertragenen AM- oder FM-Rundfunksignals vorgesehen ist, wobei zur Frequenzumsetzung (21) des mindestens einen analog übertragenen Rundfunksignals Signale verwendet werden, die von Empfängerkomponenten (17,22,26) ausgegeben werden, die zur Frequenzumsetzung des mindestens einen digital übertragenen Rundfunksignals vorgesehen sind, **dadurch gekennzeichnet, dass** das analog bei AM-Frequenzen übertragene AM- Rundfunksignal mittels eines aus einer Referenzfrequenz (f_{ref}) abgeleiteten Signals, in die Frequenz zum Empfang eines anderen Empfangsbandes, das zum Empfang des analog übertragenen FM-Rundfunksignals vorgesehen ist, umgesetzt wird und dass das aus der Referenzfrequenz abgeleitete Signal weiter in dem Signalpfad des anderen Empfangsbandes, das zum Empfang des analog übertragenen FM-Rundfunksignals vorgesehen ist, verarbeitet wird und zum Empfang des digital übertragenen Rundfunkprogramms eine doppelte Mischstufe vorgesehen ist, wobei ein oder zwei ZF-Filter integriert sind, wobei zu dem ZF-Filter (13,15,29) des Signalpfadteils, der für mehrere Empfangsfrequenzen und/oder Empfangsbänder genutzt wird, ein oder mehrere Filter (13,15, 29) gleicher oder verschiedener Bandbreite seriell oder alternativ zuschaltbar sind.

2. Vorrichtung nach Anspruch 1, **dadurch gekennzeichnet, dass** das digital übertragene Hörfunksignal auf einem oder mehreren unterschiedlichen Frequenzbändern übertragen wird.

3. Vorrichtung nach Anspruch 1 oder 2, **dadurch gekennzeichnet, dass** das Digital übertragene Hörfunksignal nach mindestens einem der Übertragungsstandards Digital Audio Broadcast, Digital Radio Mondiale, Digital Multimedia Broadcast, Digital Video Broadcast Terrestrisch übertragen wird.

4. Vorrichtung nach einem der vorhergehenden Ansprüche, **dadurch gekennzeichnet, dass** ein erstes analog übertragenes Rundfunksignal frequenzmodulierter Hörfunk , insbesondere bei Trägerfrequenzen zwischen 88 MHz und 108 MHz und ein zweites analog übertragenes Rundfunksignal amplitudenmodulierter Hörfunk, insbesondere bei Trägerfrequenzen zwischen etwa 153kHz und 26,5MHz ist.

5. Vorrichtung nach einem der vorhergehenden Ansprüche, **dadurch gekennzeichnet, dass** die Einrichtungen zum Empfang der Signale in den verschiedenen Frequenzbereichen, die typischerweise für den DAB-, FM- und AM-Empfang genutzt werden auf einem Chip (4) monolithisch integriert ist.

6. Vorrichtung nach einem der vorhergehenden Ansprüche, bei dem eines der empfangenen Signale (2,3,18,19) analoge Tonträger eines terrestrischen Fernsehsignales oder ein DVB-T Signal darstellt.

7. Vorrichtung nach einem der vorhergehenden Ansprüche, **dadurch gekennzeichnet, dass** ein analoger Zwischenfrequenzverstärker (12) vorgesehen ist, der zur Ausgabe eines der in verschiedenen Empfangsbändern empfangenen Signals vorgesehen ist, wobei die Signale gleichzeitig oder alternativ verstärkt werden.

8. Vorrichtung nach einem der vorhergehenden Ansprüche, **dadurch gekennzeichnet, dass** das ZF-Filter eines oder mehrer Signalpfade mit einer Mischstufe integriert ist.

9. Vorrichtung nach einem der vorhergehenden Ansprüche, **dadurch gekennzeichnet, dass** für mindestens zwei Mischstufen unterschiedlicher Signalpfade das Mischsignal aus einem gemeinsamen Oszillator abgeleitet wird.

10. Vorrichtung nach einem der vorhergehenden Ansprüche, **dadurch gekennzeichnet, dass** die signalverarbeitende Stufe, die die Signale des ZF-Verstärkers weiterverarbeitet, ein auf dem Chip monolithisch integrierter Analog/Digital-Wandler ist.

## Claims

1. Apparatus for receiving broadcast radio signals from two or more transmission standards which are partly broadcast in different frequency bands and which are processed in mutually independent signal paths (5 to 16; 6 to 16; 20 to 16; 23 to 16), wherein the apparatus is provided for the purpose of receiving at least one digitally transmitted broadcast radio signal and for the purpose of receiving at least one analogue-transmitted AM or FM broadcast radio signal, wherein the frequency conversion (21) of the at least one analogue-transmitted broadcast radio signal involves the use of signals which are output by receiver components (17, 22, 26) which are provided for the frequency conversion of the at least one digitally transmitted broadcast radio signal, **characterized in that** the analogue-transmitted AM broadcast radio signal, transmitted on AM frequencies, is converted by means of a signal derived from a reference frequency (f_{ref}) into the frequency for receiving another reception band, which is provided for receiving the analogue-transmitted FM broadcast radio signal, and **in that** the signal derived from the reference frequency is further processed in the signal path of the other reception band, which is provided for receiving the analogue-transmitted FM broadcast radio signal, and a dual mixing stage is provided for receiving the digitally transmitted broadcast radio programme, wherein one or two IF filters are integrated, with one or more filters (13, 15, 29) with the same or a different bandwidth being able to be connected in series or alternatively to the IF filter (13, 15, 29) of the signal path portion which is used for a plurality of reception frequencies and/or reception bands.

2. Apparatus according to Claim 1, **characterized in that** the digitally transmitted radio signal is transmitted on one or more different frequency bands.

3. Apparatus according to Claim 1 or 2, **characterized in that** the digitally transmitted radio signal is transmitted on the basis of at least one of the transmission standards Digital Audio Broadcast, Digital Radio Mondiale, Digital Multimedia Broadcast, Digital Video Broadcast Terrestrial.

4. Apparatus according to one of the preceding claims, **characterized in that** a first analogue-transmitted broadcast radio signal is frequency-modulated radio, particularly at carrier frequencies between 88 MHz and 108 MHz, and a second analogue-transmitted broadcast radio signal is amplitude-modulated radio, particularly at carrier frequencies between approximately 153 kHz and 26.5 MHz.

5. Apparatus according to one of the preceding claims, **characterized in that** the devices for receiving the signals in the various frequency ranges which are typically used for DAB, FM and AM reception are monolithically integrated on a chip (4).

6. Apparatus according to one of the preceding claims, in which one of the received signals (2, 3, 18, 19) represents analogue sound carriers for a terrestrial television signal or a DVB-T signal.

7. Apparatus according to one of the preceding claims, **characterized in that** an analogue intermediate-frequency amplifier (12) is provided which is provided for the purpose of outputting one of the signals received in various reception bands, wherein the signals are amplified simultaneously or alternatively.

8. Apparatus according to one of the preceding claims, **characterized in that** the IF filter of one or more signal paths is integrated with a mixing stage.

9. Apparatus according to one of the preceding claims, **characterized in that,** for at least two mixing stages of different signal paths, the mixed signal is derived from a common oscillator.

10. Apparatus according to one of the preceding claims, **characterized in that** the signalprocessing stage which further processes the signals from the IF amplifier is an analogue/digital converter monolithically integrated on the chip.

## Revendications

1. Dispositif de réception de signaux de radiodiffusion à deux normes de transmission ou plus, lesquels sont en partie émis dans des bandes de fréquences différentes et sont traités dans des trajets de signal (5 à 16 ; 6 à 16 ; 20 à 16 ; 23 à 16) indépendants les uns des autres, le dispositif étant prévu pour recevoir au moins un signal de radiodiffusion transmis sous forme numérique et pour recevoir au moins un signal de radiodiffusion AM ou FM transmis sous forme analogique, la conversion de fréquence (21) de l'au moins un signal de radiodiffusion transmis sous forme analogique s'effectuant en utilisant des signaux qui sont délivrés par les composantes du récepteur (17, 22, 26) qui sont prévues pour la conversion de fréquence de l'au moins un signal de radiodiffusion transmis sous forme numérique, **caractérisé en ce que** le signal de radiodiffusion AM analogique transmis à des fréquences AM est converti au moyen d'un signal dérivé d'une fréquence de référence (f_{ref}) en la fréquence requise pour la réception d'une autre bande de réception qui est prévue pour recevoir le signal de radiodiffusion FM transmis sous forme analogique et que le signal dérivé de la fréquence de référence est ensuite traité dans le trajet du signal de l'autre bande de réception qui est prévue pour la réception du signal de radiodiffusion FM transmis sous forme analogique et un double étage mélangeur est prévu pour la réception du programme radiodiffusé transmis sous forme numérique, un ou deux filtres FI étant intégrés, un ou plusieurs filtres (13, 15, 29) ayant des largeurs de bande égales ou différentes pouvant être connectés en série ou en alternance au filtre FI (13, 15, 29) de la partie du trajet du signal qui est utilisée pour plusieurs fréquences de réception et/ou bandes de réception.

2. Dispositif selon la revendication 1, **caractérisé en ce que** le signal de radiodiffusion sonore transmis sous forme numérique est transmis sur une ou plusieurs bandes de fréquences différentes.

3. Dispositif selon la revendication 1 ou 2, **caractérisé en ce que** le signal de radiodiffusion sonore transmis sous forme numérique est transmis par voie terrestre selon au moins l'une des normes de transmission parmi la Radiodiffusion audionumérique (DAB), le Système mondial de radiodiffusion numérique, la Diffusion multimédia numérique (DMB), la Vidéodiffusion audionumérique (DVB).

4. Dispositif selon l'une des revendications précédentes, **caractérisé en ce qu'**un premier signal de radiodiffusion transmis sous forme analogique est un signal de radiodiffusion sonore modulé en fréquence, notamment à des fréquences porteuses entre 88 MHz et 108 MHz, et un deuxième signal de radiodiffusion transmis sous forme analogique est un signal de radiodiffusion sonore modulé en amplitude, notamment à des fréquences porteuses entre 153 kHz et 26,5 MHz.

5. Dispositif selon l'une des revendications précédentes, **caractérisé en ce que** les dispositifs de réception des signaux dans les différentes plages de fréquences qui sont généralement utilisées pour la réception DAB, FM et AM sont intégrés de manière monolithique sur une puce (4).

6. Dispositif selon l'une des revendications précédentes, **caractérisé en ce que** l'un des signaux reçus (2, 3, 18, 19) représente la porteuse sonore analogique d'un signal de télévision terrestre ou d'un signal DVB-T.

7. Dispositif selon l'une des revendications précédentes, **caractérisé en ce qu'**il est prévu un compteur de fréquence intermédiaire analogique (12) qui est conçu pour délivrer l'un des signaux reçus dans différentes bandes de réception, les signaux étant amplifiés simultanément ou en alternance.

8. Dispositif selon l'une des revendications précédentes, **caractérisé en ce que** le filtre FI d'un ou de plusieurs trajets du signal est intégré avec un étage mélangeur.

9. Dispositif selon l'une des revendications précédentes, **caractérisé en ce que**, pour au moins deux étages mélangeurs de trajets de signal différents, le signal mélangé est dérivé d'un oscillateur commun.

10. Dispositif selon l'une des revendications précédentes, **caractérisé en ce que** l'étage de traitement du signal qui réalise le traitement postérieur des signaux de l'amplificateur FI est un convertisseur analogique/numérique intégré de manière monolithique sur la puce.
